# EUROPEAN PATENT SPECIFICATION

(11) **EP 2 663 239 B1**
(45) Date of publication and mention of the grant of the patent: **26.08.2015**
(21) Application number: 12701168.2
(22) Date of filing: 05.01.2012
(51) Int. Cl.: A61B 17/00, A61B 19/00

(54) **DETACHMENT TOOL FOR DECOUPLING A SHAPE SENSOR FROM AN IMPLANTABLE DEVICE**
ABLÖSUNGSWERKZEUG ZUR ABKOPPLUNG EINES FORMSENSORS VON EINER IMPLANTIERBAREN VORRICHTUNG
OUTIL DE DÉTACHEMENT POUR DÉCOUPLER UN CAPTEUR DE FORME D'UN DISPOSITIF IMPLANTABLE

(30) Priority: 13.01.2011 US 201161432222 P
(43) Date of publication of application: 20.11.2013
(73) Proprietor: Koninklijke Philips N.V., 5656 AE Eindhoven (NL)
(72) Inventor: MANZKE, Robert, NL-5656 AE Eindhoven (NL); GUTIERREZ, Luis, Felipe, NL-5656 AE Eindhoven (NL); CHAN, Raymond, NL-5656 AE Eindhoven (NL)
(74) Representative: Steffen, Thomas
(86) International application number: PCT/IB2012/050048
(87) International publication number: WO 2012/095760

(56) References cited:
- EP-A1- 1 738 693
- EP-A1- 2 233 902
- EP-A1- 2 260 784
- WO-A2-03/032818
- WO-A2-2008/131303
- US-A1- 2005 154 417

## Description

The present invention generally relates to a deployment tool for interfacing an implantable device with an anatomical structure (e.g., biological tissue and organs). The present invention specifically relates to a deployment tool having a shape sensor for guiding an implantable device to an interface position with respect to the anatomical structure and a detachment tool for decoupling the shape sensor from the deployed implantable device.

Recent medical research has led to various innovative minimally invasive deployed implantable devices. Examples of such deployments include a deployment of left atrial occlusion devices, filter devices, physiological monitoring devices, septal defect repair devices, valve replacement devices, cardiac resynchronization therapy devices, pacing devices, stimulating devices, and neuroendovascular repair devices.

EP 1 738693 A1 discloses a vascular occlusion device deployment system for placing an occlusion device at a preselected site within the vasculature of a patient. The deployment system employs light energy transferred through an optical fiber to sever a securing filament attaching the occlusion device to the deployment system, thereby releasing the occlusion device at the preselected location.

WO 2008/131303 A2 discloses optical fibers with Bragg gratings that are configured to provide real-time feedback for sensing and determining the fibers' dynamic shape, positions, temperatures, and stress or strain along an elongate steerable instrument.

Clinical procedures for implantation of such devices are often times complex and therefore require interventional guidance technologies. However, most implantable devices do not incorporate a guiding sensor of any type, particularly a shape sensor for guidance during deployment of the implantable devices. Furthermore, implantable devices that do incorporate a guiding sensor only utilize low-power, wireless sensor types (e.g., wireless pressure sensors incorporated into stents).

The present invention utilizes a shape sensor in the deployment of an implantable device to take advantage of the imaging, sensing and tracking benefits provided by shape sensors. Specifically, the present invention provides a shape sensor having an implantable segment extending into the implantable device to allow for imaging, sensing and tracking during deployment of the implantable device. After deployment, the present invention provides a detachment tool for decoupling a portion or an entirety of the implantable segment from the shape sensor.

One form of the present invention is a deployment device for interfacing an implantable device with an anatomical structure. For example, the implantable device may be designed to serve as a replacement of a missing anatomical structure, support a damaged anatomical structure, or improve upon an existing anatomical structure. As such, the deployment device is operated to interface the implantable device with the anatomical structure and/or an adjacent anatomical structure in a manner suitable for the purpose of the implantable device.

The deployment device employs a sheath, a shape sensor and a detachment tool. The sheath includes a deployment section for deploying the implantable device to an interface position relative to the anatomical structure, and an implantable section for coupling the deployment section to the implantable device. The shape sensor includes a deployment segment extending partially or completely through the deployment section of the sheath, and an implantable segment extending attached (i.e., adjoined, coupled or integrated) to the deployment segment and extending partially or completely through the implantable section of the sheath. The detachment tool is disposed relative to the implantable section of the sheath and in operation, the detachment tool may be used to detach a portion or an entirety of the implantable segment of the shape sensor from the deployment segment of the shape sensor.

A second form of the present invention is a deployment system employing the aforementioned deployment device and a shape sensor monitor for sensing a shape of the shape sensor.

The foregoing forms and other forms of the present invention as well as various features and advantages of the present invention will become further apparent from the following detailed description of various embodiments of the present invention read in conjunction with the accompanying drawings. The detailed description and drawings are merely illustrative of the present invention rather than limiting, the scope of the present invention being defined by the appended claims and equivalents thereof.
FIGS. 1 and 2 illustrate an exemplary embodiment of an implantable device deployment system in accordance with the present invention.
FIGS. 3A and 3B illustrate a first exemplary embodiment of a detachment tool in accordance with the present invention.
FIGS. 4A and 4B illustrate a second exemplary embodiment of a detachment tool in accordance with the present invention.
FIGS. 5A and 5B illustrate a third exemplary embodiment of a detachment tool in accordance with the present invention.
FIGS. 6A and 6B illustrate a fourth embodiment of a detachment tool in accordance with the present invention.

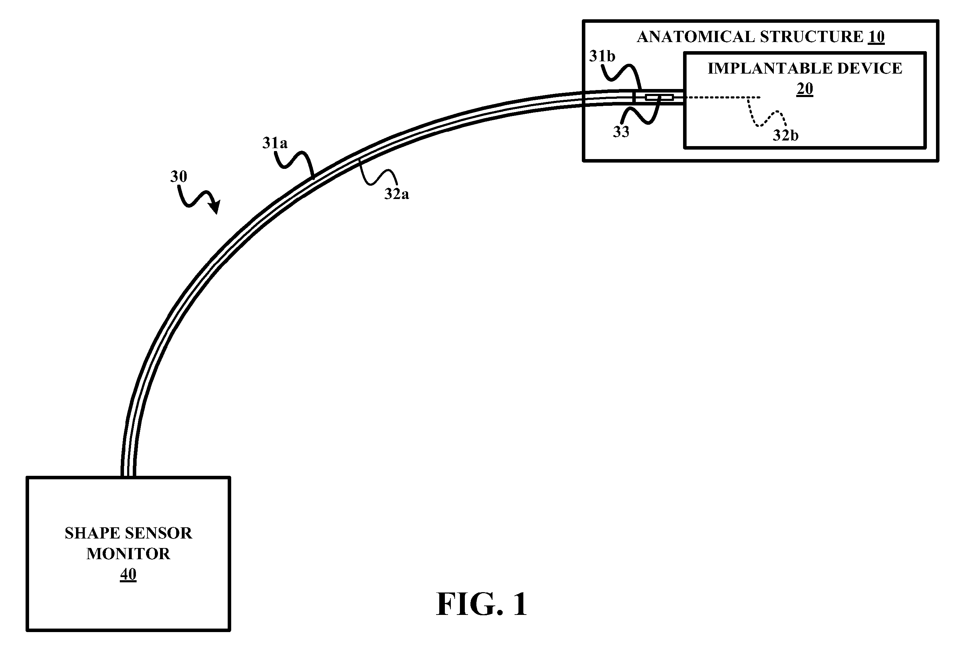

FIG. 1 illustrates a deployment tool 30 of the present invention for interfacing an implantable device 20 within an anatomical structure 10 (e.g., biological tissue and organs). For example, implantable device 20 serve as a replacement of an anatomical structure that was adjacent to anatomical structure 10 and deployment tool 30 may be operated to interface implantable device 20 with anatomical structure 10 in a manner that replaces the missing anatomical structure. Also by example, implantable device 20 may serve to support any damage to anatomical structure 10 and/or to improve upon anatomical structure 10, and deployment tool 30 may be operated to interface implantable device 20 with anatomical structure 10 in a manner that supports and/or improves upon anatomical structure 10.

To this end, deployment tool 30 employs a sheath 31, a shape sensor 32 and a detachment tool 33.

Sheath 31 has a deployment section 31a for deploying the implantable device 20 to an interface position relative to the anatomical structure 10. Sheath 31 further has an implantable section 31b attached (i.e., adjoined, coupled or integrated) to deployment section 21a for coupling deployment section 31 a to implantable device 20. In practice, sheath 31 may have a tubular structure as shown in FIG. 1. However, the present invention does not impose any restrictions or limitations to the structural configuration of sheath 31.

Shape sensor 32 has a deployment segment 32a and an implantable section 32b for guiding implantable device 20 to the interface position via a tracking of the shape sensor 32 as known in the art. Deployment segment 32a extends partially or completely through deployment section 31a of sheath 31, and implantable section 32b is attached (i.e., adjoined, coupled or integrated) to deployment segment 32a and extends partially or completely through implantable section 31b of sheath 31. Implantable section 32b may extend into implantable device 20 as shown in FIG. 1 or may not. In practice, shape sensor 32 may have an elongated structure as shown in FIG. 1. However, the present invention does not impose any restrictions or limitations to the structural configuration of shape sensor 32.

In one embodiment, shape sensor 32 may be an optical fiber composed of a flexible optically transparent glass or plastic fiber incorporating an array of fiber Bragg gratings integrated along a length of the fiber as known in the art; or a flexible optically transparent glass or plastic fiber having naturally variations in its optic refractive index occurring along a length of the fiber as known in the art; or a flexible optically transparent glass or plastic fiber having variations along the length of the fiber using back scattering, optical fiber force sensing, fiber location sensors or Rayleigh scattering.

In another embodiment, shape sensor 32 is a wired tether having an embedded array of shape sensing elements (e.g., electromagnetic coils).

A shape sensor monitor 40 is provided for monitoring the implantation of implantable device 20 into anatomical structure 10 via shape sensor 32 as known in the art. As such, the structural configuration of shape sensor monitor 40 in practice is dependent upon the type of shape sensor 32 being utilized in the implantation process. Additionally, the means for guiding implantable device 20 via deployment tool 30 during the implantation process is dependent upon various factors, such as, for example, the particular implantation process and the actual structural configuration of deployment tool 30.

Upon implantable device 20 being implanted within the anatomical structure 10, detachment tool 33 is activated for decoupling a portion or an entirety of implantable segment 32b of shape sensor 30 from deployment segment 32a of shape sensor 30. Subsequently, deployment section 31a of sheath 31 is detached from implantable section 31b of sheath 31 as known in the art, or alternatively, implantable section 31b of sheath 31 is detached from implantable device 20 as known in the art.

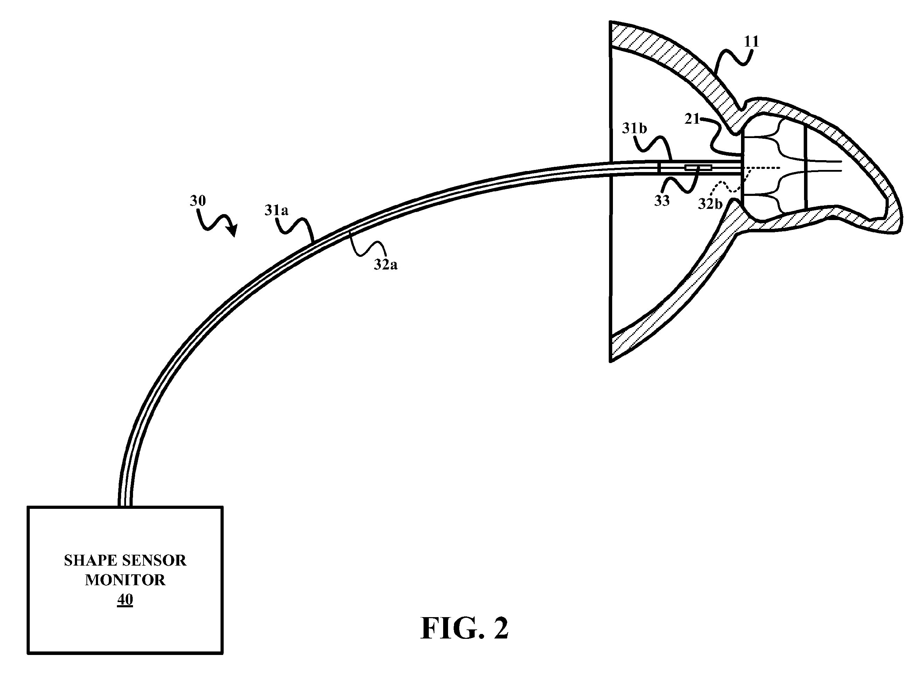

To facilitate a further understanding of the deployment tool 30, FIG. 2 illustrates a use of deployment tool 30 for a left atrial appendage occlusion process involving an implantation of a left atrial occlusion device 21 within a left atrial of a heart 11. As shown, implantable section 31b of sheath 31 is coupled to implantable device 21 and implantable segment 32b of shape sensor 32 extends into implantable device 21. Upon implantable device 21 being implanted within the left atrium of heart 11, detachment tool 33 is activated for detaching a portion or an entirety of implantable segment 32b of shape sensor 32 from deployment segment 32a of shape sensor 32. Subsequently, deployment section 31a of sheath 31 is detached from implantable section 31b of sheath 31 as known in the art, or alternatively, implantable section 31b of sheath 31 is detached from implantable device 21 as known in the art.

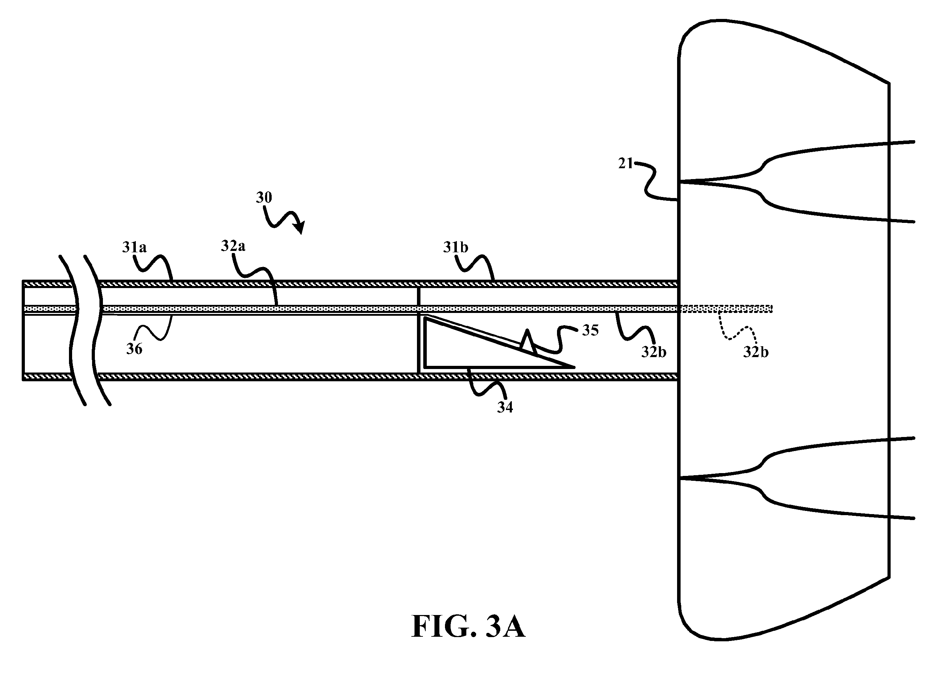

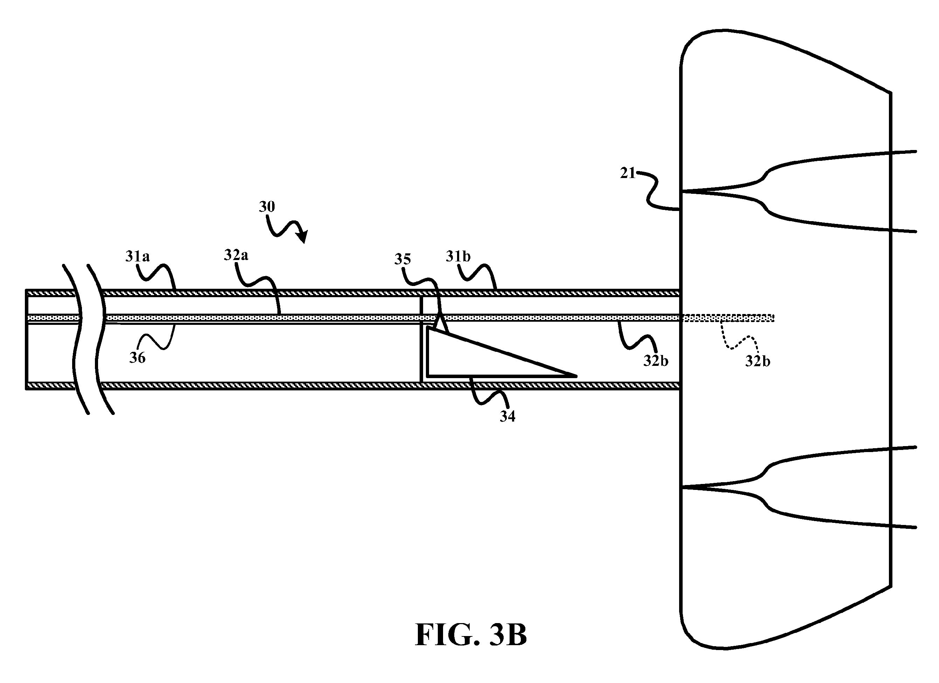

In one embodiment of detachment tool 33 as shown in FIGS. 3A and 3B, a base wedge 34 is securely disposed within implantable section 31b of sheath 31, and a clipping wedge 35 is slidable along base wedge 34. In a deployment position as shown in FIG. 3A, clipping wedge 35 is positioned along a bottom portion of base wedge 34 whereby clipping wedge 35 is spaced from implantable segment 32b of shape sensor 32. In a detachment position as shown in FIG. 3B, clipping wedge 35 has been slid along base wedge 34 via a wire 36 to detach a portion of implantable segment 32b of shape sensor 32 that remains in implantable device 21.

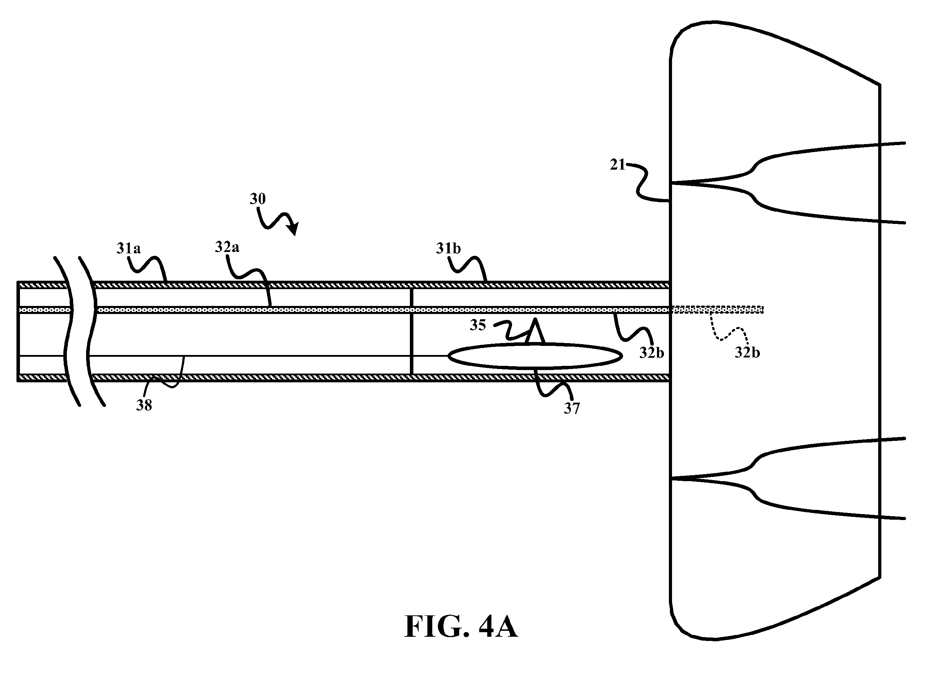

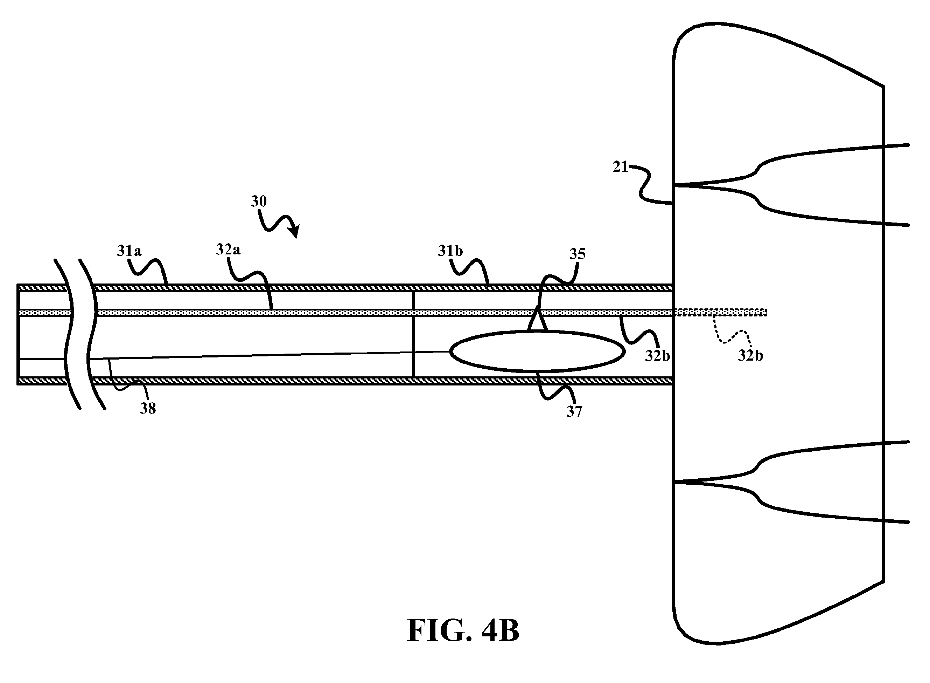

In a second embodiment of detachment tool 33 as shown in FIGS. 4A and 4B, a cutting balloon includes an inflatable balloon 37 securely disposed within implantable section 31b of sheath 31, and clipping wedge 35 positioned on top of balloon 37. In a deployment position as shown in FIG. 4A, balloon 37 is deflated whereby clipping wedge 35 is spaced from implantable segment 32b of shape sensor 32. In a detachment position as shown in FIG. 3B, balloon 37 has been inflated via a channel 38 to detach a portion of implantable segment 32b of shape sensor 32 that remains in implantable device 21.

In a third embodiment of detachment tool 33, a sensor detacher encloses or is integrated with shape sensor 32. For purposes of the present invention, the sensor detacher is broadly defined as any article for detaching a portion or an entirety of implantable segment 32b of shape sensor 32 from deployment segment 32a of shape sensor 32 in response to an external stimulus applied to or coupled into shape sensor 32.

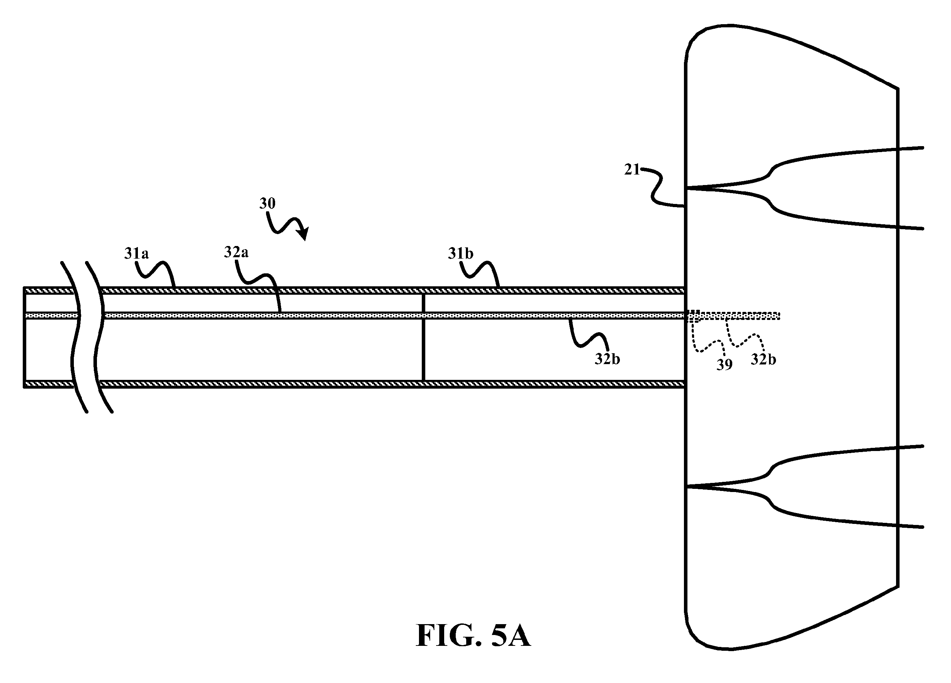

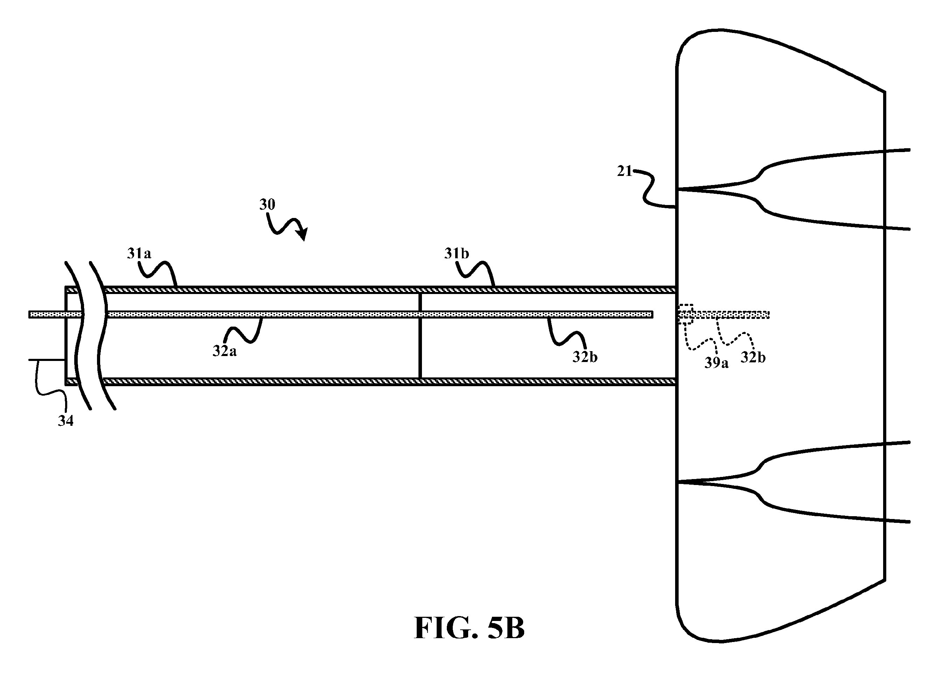

For example, as shown in FIGS. 5A and 5B, a sensor detacher 39a in the form of a mechanical clip is to implantable section 31b of sheath 31 via a seal (not shown) and extends into implantable device 21. In a deployment mode as shown in FIG. 5A, a lateral force opposing sensor detacher 39a is not applied to shape sensor 32 whereby the segments 32a, 32b of shape sensor 32 remain attached. In a detachment mode as shown in FIG. 5B, a lateral force opposing sensor detacher 39a is applied to shape sensor 32 to detach a portion of implantable segment 32b of shape sensor 32 that remains in implantable device 21.

By a second example, sensor detacher 39a is an adhesive for bonding segments 32a and 32b of shape sensor 32 whereby a lateral force opposing sensor detacher 39a is applied to shape sensor 32 to detach a portion of implantable segment 32b from deployment segment 32a.

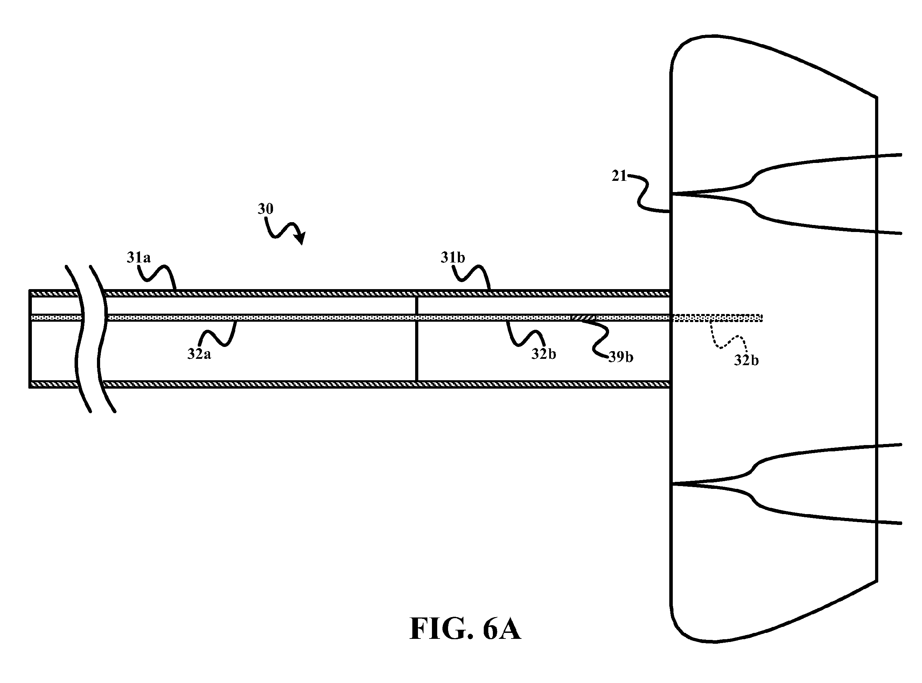

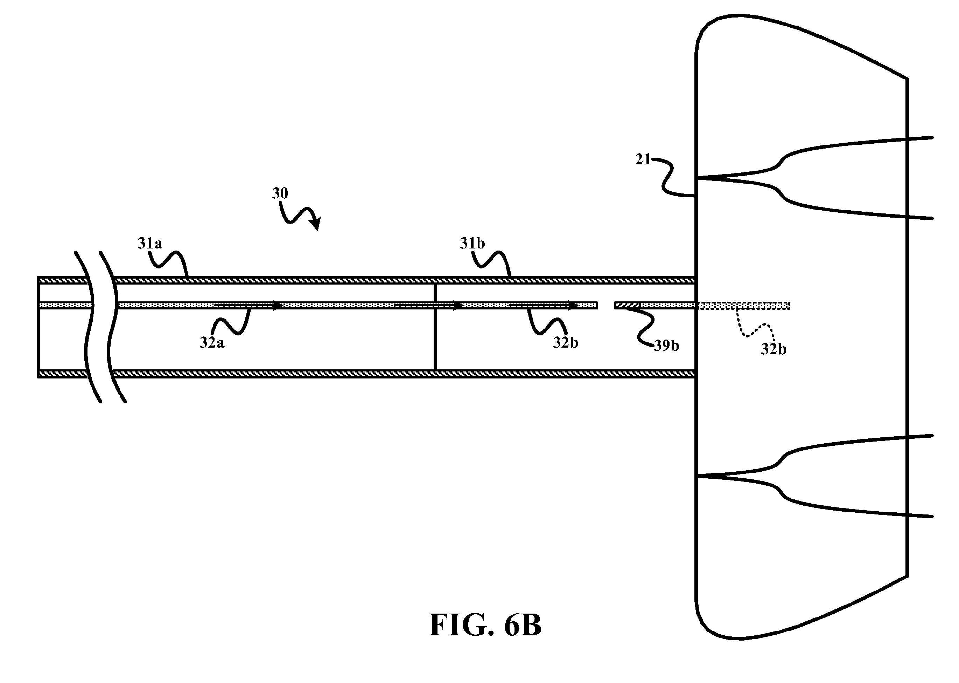

By a third example, as shown in FIGS. 6A and 6B, a sensor detacher 39b is located on implantable segment 32b of shape sensor 32 within on implantable section 31b of sheath 31 as shown or within implantable device 21. In this example, shape sensor 32 is an optical fiber and sensor detacher 39b is a rated break point for detaching segments 32a, 32b of shape sensor 32 if laser light of a specified wavelength exceeding a specified power level is coupled into shape sensor 32 as known in the art. As such, in a deployment mode as shown in FIG. 6A, the laser light is not coupled into shape sensor 32 whereby segments 32a and 32b of shape sensor 32 remain attached. In a detachment mode as shown in FIG. 6B, the laser light is coupled into shape sensor 32 as shown by the arrows to thereby detach a portion of implantable segment 32b of shape sensor 32 that remains in implantable device 21.

By a fourth example, sensor detacher 39b may be a magnetic coupler responsive to an electrical signal of a specified amplitude for detaching segments 32a, 32b of shape sensor 32. In particular, the magnetic coupler employs a magnet and an electromagnetic. In a deployment mode as shown in FIG. 6A, an electrical signal of a specified amplitude is coupled into shape sensor 32 to magnetically activate sensor detacher 39b whereby segments 32a and 32b of shape sensor 32 are attached. In a detachment mode as shown in FIG. 6B, the electric signal is attenuated or decoupled from shape sensor 32 to magnetically deactivate sensor detacher 39b whereby a portion of implantable segment 32b detaches from deployment segment 32a.

Referring to FIGS. 1-6, those having ordinary skill in the art will appreciate the various benefits of the present invention including, but not limited to, a deployment tool for guiding an implantable device to an anatomical structure via a shape sensor with the means to decouple a portion of the shape sensor from the implantable device.

While various exemplary embodiments of the present invention have been illustrated and described, it will be understood by those skilled in the art that the exemplary embodiments of the present invention as described herein are illustrative, and various changes and modifications may be made and equivalents may be substituted for elements thereof without departing from the scope of the present invention. In addition, many modifications may be made to adapt the teachings of the present invention without departing from its scope. Therefore, it is intended that the present invention not be limited to the particular embodiments disclosed as the best mode contemplated for carrying out the present invention, but that the present invention includes all embodiments falling within the scope of the appended claims.

## Claims

1. A deployment device (30) for interfacing an implantable device (20) with an anatomical structure (10), the deployment device (30) comprising:
a sheath (31) including
a deployment section (31a) for deploying the implantable device (20) to an interface position relative to the anatomical structure (10), and
an implantable section (31b) for coupling the deployment section (31a) to the implantable device (20);
a detachment tool (33);
**characterized in that** the device further comprises
a shape sensor (32) for guiding the implantable device (20) to the interface position, the shape sensor (32) including
a deployment segment (32a) extending at least partially through the deployment section (31a) of the sheath (31), and
an implantable segment (32b) attached to the deployment segment (32a) and extending at least partially through the implantable section (31b) of the sheath (31); and
wherein said detachment tool (33) is disposed relative to the implantable section (31b) of the sheath (31), the detachment tool (33) being operable to detach at least a portion of the implantable segment (32b) of the shape sensor (32) from the deployment segment (32a) of the shape sensor (32).

2. The deployment device (30) of claim 1, wherein the shape sensor (32) is an optical fiber.

3. The deployment device (30) of claim 1, wherein the shape sensor (32) is a wired tether including an array of shape sensing elements.

4. The deployment device (30) of claim 1, wherein the implantable segment (32b) extends through the implantable section (31b) of the sheath (31) into the implantable device (20).

5. The deployment device (30) of claim 1, wherein the detachment tool (33) includes:
a base wedge (34) disposed within the implantable section (31b) of the sheath (31); and
a clipping wedge (35) slidable along the base wedge (34), wherein the clipping wedge (35) is operable to detach at least a portion of the implantable segment (32b) of the shape sensor (32) from the deployment segment (32a) of the shape sensor (32) in response to the clipping wedge (35) being slid along the base wedge (34) in a direction of the implantable segment (32b).

6. The deployment device (30) of claim 1, wherein the detachment tool (33) is a cutting balloon including:
an inflatable balloon (37) disposed within the implantable section (31b) of the sheath (31); and
a clipping wedge (35) positioned on the balloon (37), wherein the clipping wedge (35) is operable to detach at least a portion of the implantable segment (32b) of the shape sensor (32) from the deployment segment (32a) of the shape sensor (32) in response to the balloon (37) being inflated.

7. The deployment device (30) of claim 1, wherein the detachment tool (33) includes a sensor detacher (39a) operable to detach at least a portion of the implantable segment (32b) of the shape sensor (32) from the deployment segment (32a) of the shape sensor (32) in response to a lateral force being applied to shape sensor (32) in a direction at least partially opposing the sensor detacher (39a).

8. The deployment device (30) of claim 1, wherein the detachment tool (33) includes a sensor detacher (39b) operable to detach at least a portion of the implantable segment (32b) of the shape sensor (32) from the deployment segment (32a) of the shape sensor (32) in response to a laser light of a specified wavelength and power being coupled into the shape sensor (32).

9. A deployment system for interfacing an implantable device (20) with an anatomical structure (10), the deployment system comprising:
the deployment device (30) according to claim 1 and
a shape sensor monitor (40) for sensing a shape of the shape sensor (32) of the deployment device of claim 1.

10. The deployment system of claim 9, wherein the shape sensor (32) is an optical fiber.

11. The deployment system of claim 9, wherein the shape sensor (32) is a wired tether including an array of shape sensing elements.

12. The deployment system of claim 9, wherein the implantable segment (32b) of the deployment device of claim 1 extends through the implantable section (31b) of the sheath (31) into the implantable device (20).

13. The deployment system of claim 9, wherein the detachment tool (33) of the deployment device of claim 1 includes a sensor detacher (39b) operable to detach at least a portion of the implantable segment (32b) of the shape sensor (32) from the deployment segment (32a) of the shape sensor (32) in response to a laser light of a specified wavelength and power being coupled into the shape sensor (32).

14. The deployment system of claim 9, wherein the detachment tool (33) of the device of claim 1 includes a sensor detacher (39b) operable to detach at least a portion of the implantable segment (32b) of the shape sensor (32) from the deployment segment (32a) of the shape sensor (32) in response to a specified amplitude of an electric signal coupled into the shape sensor (32).

15. The deployment system of claim 9, wherein the implantable device (20) is selected from a group including a left atrial occlusion device, a filter device, a physiological monitoring device, a septal defect repair device, a valve replacement device, a cardiac resynchronization therapy device, a pacing device, a stimulating device, and a neuroendovascular repair device.

## Patentansprüche

1. Einsetzvorrichtung (30) zum Verbinden einer implantierbaren Vorrichtung (20) mit einer anatomischen Struktur (10), wobei die Einsetzvorrichtung (30) Folgendes umfasst:
einen Mantel (31) mit
einem Einsetzabschnitt (31 a) zum Einsetzen der implantierbaren Vorrichtung (20) in eine Verbindungsposition in Bezug auf die anatomische Struktur (10), und
einen implantierbaren Abschnitt (31b) zum Koppeln des Einsetzabschnitts (31a) mit der implantierbaren Vorrichtung (20);
ein Ablösungswerkzeug (33);
**dadurch gekennzeichnet, dass** die Vorrichtung weiterhin Folgendes umfasst:
einen Formsensor (32) zum Führen der implantierbaren Vorrichtung (20) zu der Verbindungsposition, wobei der Formsensor (32) Folgendes umfasst:
ein Einsetzsegment (32a), das zumindest teilweise durch den Einsetzabschnitt (31a) des Mantels (31) verläuft, und
ein implantierbares Segment (32b), das an dem Einsetzsegment (32a) angebracht ist und sich zumindest teilweise durch den implantierbaren Abschnitt (31b) des Mantels (31) erstreckt; und
wobei das genannte Ablösungswerkzeug (33) relativ zu dem implantierbaren Abschnitt (31b) des Mantels (31) angeordnet ist, wobei das Ablösungswerkzeug (33) bedienbar ist, um zumindest einen Teil des implantierbaren Segments (32b) des Formsensors (32) von dem Einsetzsegment (32a) des Formsensors (32) abzulösen.

2. Einsetzvorrichtung (30) nach Anspruch 1, wobei der Formsensor (32) eine optische Faser ist.

3. Einsetzvorrichtung (30) nach Anspruch 1, wobei der Formsensor (32) ein verdrahteter Gurt mit einem Array aus Formsensorelementen ist.

4. Einsetzvorrichtung (30) nach Anspruch 1, wobei sich das implantierbare Segment (32b) durch den implantierbaren Abschnitt (31b) des Mantels (31) in die implantierbare Vorrichtung (20) hinein erstreckt.

5. Einsetzvorrichtung (30) nach Anspruch 1, wobei das Ablösungswerkzeug (33) Folgendes umfasst:
einen Basiskeil (34), der innerhalb des implantierbaren Abschnitts (31b) des Mantels (31) angeordnet ist; und
einen Schnittkeil (35), der am Basiskeil (34) entlang verschiebbar ist, wobei der Schnittkeil (35) dazu dient, mindestens einen Teil des implantierbaren Segments (32b) des Formsensors (32) von dem Einsetzsegment (32a) des Formsensors (32) abzulösen, wenn der Schnittkeil (35) in Richtung des implantierbaren Segments (32b) an dem Basiskeil (34) entlang geschoben wird,

6. Einsetzvorrichtung (30) nach Anspruch 1, wobei das Ablösungswerkzeug (33) ein Schneidballon ist, der Folgendes umfasst:
einen inflatierbaren Ballon (37), der innerhalb des implantierbaren Abschnitts (31b) des Mantels (31) angeordnet ist; und
einen Schnittkeil (35), der auf dem Ballon (37) positioniert ist, wobei der Schnittkeil (35) dazu dient, mindestens einen Teil des implantierbaren Segments (32b) des Formsensors (32) von dem Einsetzsegment (32a) des Formsensors (32) abzulösen, wenn der Ballon (37) inflatiert wird.

7. Einsetzvorrichtung (30) nach Anspruch 1, wobei das Ablösungswerkzeug (33) einen Sensorablöser (39a) umfasst, der dazu dient, mindestens einen Teil des implantierbaren Segments (32b) des Formsensors (32) von dem Einsetzsegment (32a) des Formsensors (32) abzulösen, wenn eine seitliche Kraft auf den Formsensor (32) in einer Richtung ausgeübt wird, die zumindest teilweise dem Sensorablöser (39a) entgegengesetzt ist.

8. Einsetzvorrichtung (30) nach Anspruch 1, wobei das Ablösungswerkzeug (33) einen Sensorablöser (39b) umfasst, der dazu dient, mindestens einen Teil des implantierbaren Segments (32b) des Formsensors (32) von dem Einsetzsegment (32a) des Formsensors (32) abzulösen, wenn ein Laserlicht mit einer bestimmten Wellenlänge und Leistung in den Formsensor (32) eingekoppelt wird.

9. Einsetzsystem zum Verbinden einer implantierbaren Vorrichtung (20) mit einer anatomischen Struktur (10), wobei das Einsetzsystem Folgendes umfasst:
die Einsetzvorrichtung (30) nach Anspruch 1 und
einen Formsensormonitor (40) zum Erfassen der Form des Formsensors (32) der Einsetzvorrichtung aus Anspruch 1.

10. Einsetzsystem nach Anspruch 9, wobei der Formsensor (32) eine optische Faser ist.

11. Einsetzsystem nach Anspruch 9, wobei der Formsensor (32) ein verdrahteter Gurt mit einem Array aus Formsensorelementen ist.

12. Einsetzsystem nach Anspruch 9, wobei sich das implantierbare Segment (32b) der Einsetzvorrichtung nach Anspruch 1 durch den implantierbaren Abschnitt (31b) des Mantels (31) in die implantierbare Vorrichtung (20) hinein erstreckt.

13. Einsetzsystem nach Anspruch 9, wobei das Ablösungswerkzeug (33) der Einsetzvorrichtung nach Anspruch 1 einen Sensorablöser (39b) umfasst, der dazu dient, mindestens einen Teil des implantierbaren Segments (32b) des Formsensors (32) von dem Einsetzsegment (32a) des Formsensors (32) abzulösen, wenn ein Laserlicht mit einer bestimmten Wellenlänge und Leistung in den Formsensor (32) eingekoppelt wird.

14. Einsetzsystem nach Anspruch 9, wobei das Ablösungswerkzeug (33) der Einsetzvorrichtung nach Anspruch 1 einen Sensorablöser (39b) umfasst, der dazu dient, mindestens einen Teil des implantierbaren Segments (32b) des Formsensors (32) von dem Einsetzsegment (32a) des Formsensors (32) zu lösen, wenn ein elektrisches Signal mit einer bestimmten Amplitude in den Formsensor (32) eingekoppelt wird.

15. Einsetzsystem nach Anspruch 9, wobei die implantierbare Vorrichtung (20) ausgewählt wird aus einer Gruppe bestehend aus einer Vorrichtung für linksatriale Okklusion, einer Filtervorrichtung, einer Vorrichtung zur Überwachung von physiologischen Parametern, einer Vorrichtung zur Reparatur von Septumdefekten, einer Vorrichtung zur Klappenreparatur, einer Vorrichtung zur kardialen Resynchronisationstherapie, einer Schrittmachervorrichtung, einer Stimulationsvorrichtung und einer Vorrichtung zur neuroendovaskulären Reparatur.

## Revendications

1. Dispositif de déploiement (30) pour l'interfaçage d'un dispositif implantable (20) avec une structure anatomique (10), le dispositif de déploiement (30) comprenant :
une gaine (31) comprenant :
une section de déploiement (31a) pour le déploiement du dispositif implantable (20) dans une position d'interface par rapport à la structure anatomique (10) et
une section implantable (31b) pour le couplage de la section de déploiement (31a) au dispositif implantable (20) ;
un outil de détachement (33) ;
**caractérisé en ce que** le dispositif comprend en outre
un capteur de forme (32) pour le guidage du dispositif implantable (20) dans la position d'interface, le capteur de forme (32) comprenant :
un segment de déploiement (32a) s'étendant au moins en partie au travers de la section de déploiement (31a) de la gaine (31) et
un segment implantable (32b) attaché au segment de déploiement (32a) et s'étendant au moins en partie au travers de la section implantable (31b) de la gaine (31) ; et
dans lequel ledit outil de détachement (33) est agencé par rapport à la section implantable (31b) de la gaine (31), l'outil de détachement (33) étant actionnable pour détacher au moins une partie du segment implantable (32b) du capteur de forme (32), du segment de déploiement (32a) du capteur de forme (32).

2. Dispositif de déploiement (30) selon la revendication 1, dans lequel le capteur de forme (32) est une fibre optique.

3. Dispositif de déploiement (30) selon la revendication 1, dans lequel le capteur de forme (32) est une attache câblée incluant un agencement d'éléments de détection de forme.

4. Dispositif de déploiement (30) selon la revendication 1, dans lequel le segment implantable (32b) s'étend au travers de la section implantable (31b) de la gaine (31) dans le dispositif implantable (20).

5. Dispositif de déploiement (30) selon la revendication 1, dans lequel l'outil de détachement (33) comprend :
un coin de base (34) agencé dans la section implantable (31b) de la gaine (31) ; et
un coin de serrage (35) pouvant glisser le long du coin de base (34), dans lequel le coin de serrage (35) est actionnable pour détacher au moins une partie du segment implantable (32b) du capteur de forme (32), du segment de déploiement (32a) du capteur de forme (32) en réponse au glissement du coin de serrage (35) le long du coin de base (34) dans une direction du segment implantable (32b).

6. Dispositif de déploiement (30) selon la revendication 1, dans lequel l'outil de détachement (33) est un ballonnet de coupe incluant :
un ballonnet gonflable (37) agencé dans la section implantable (31b) de la gaine (31) ; et
un coin de serrage (35) positionné sur le ballonnet (37), dans lequel le coin de serrage (35) est actionnable pour détacher au moins une partie du segment implantable (32b) du capteur de forme (32), du segment de déploiement (32a) du capteur de forme (32) en réponse au gonflage du ballonnet (37).

7. Dispositif de déploiement (30) selon la revendication 1, dans lequel l'outil de détachement (33) inclut un détacheur de capteur (39a) actionnable pour détacher au moins une partie du segment implantable (32b) du capteur de forme (32), du système de déploiement (32a) du capteur de forme (32) en réponse à l'application d'une force latérale au capteur de forme (32) dans une direction au moins partiellement opposée au détacheur de capteur (39a).

8. Dispositif de déploiement (30) selon la revendication 1, dans lequel l'outil de détachement (33) inclut un détacheur de capteur (39b) actionnable pour détacher au moins une partie du segment implantable (32b) du capteur de forme (32), du segment de déploiement (32a) du capteur de forme (32) en réponse au couplage d'une lumière de laser d'une longueur d'onde et d'une puissance spécifiées dans le capteur de forme (32).

9. Système de déploiement pour l'interfaçage d'un dispositif implantable (20) avec une structure anatomique (10), le système de déploiement comprenant :
le dispositif de déploiement (30) selon la revendication 1 et
un moniteur de capteur de forme (40) pour la détection d'une forme du capteur de forme (32) du dispositif de déploiement selon la revendication 1.

10. Système de déploiement selon la revendication 9, dans lequel le capteur de forme (32) est une fibre optique.

11. Système de déploiement selon la revendication 9, dans lequel le capteur de forme (32) est une attache câblée incluant un agencement d'éléments de détection de forme.

12. Système de déploiement selon la revendication 9, dans lequel le segment implantable (32b) du dispositif de déploiement selon la revendication 1 s'étend au travers de la section implantable (31b) de la gaine (31) dans le dispositif implantable (20).

13. Système de déploiement selon la revendication 9, dans lequel l'outil de détachement (33) du dispositif de déploiement selon la revendication 1 inclut un détacheur de capteur (39b) actionnable pour détacher au moins une partie du segment implantable (32b) du capteur de forme (32), du segment de déploiement (32a) du capteur de forme (32) en réponse au couplage d'une lumière de laser d'une longueur d'onde et d'une puissance spécifiées dans le capteur de forme (32).

14. Système de déploiement selon la revendication 9, dans lequel l'outil de détachement (33) du dispositif selon la revendication 1 inclut un détacheur de capteur (39b) actionnable pour détacher au moins une partie du segment implantable (32b) du capteur de forme (32), du segment de déploiement (32a) du capteur de forme (32) en réponse au couplage d'une amplitude spécifiée d'un signal électrique dans le capteur de forme (32).

15. Système de déploiement selon la revendication 9, dans lequel le dispositif implantable (20) est sélectionné dans un groupe incluant un dispositif d'occlusion auriculaire gauche, un dispositif de filtre, un dispositif de surveillance physiologique, un dispositif de réparation de défaut du septum, un dispositif de remplacement de valve, un dispositif de traitement de resynchronisation cardiaque, un dispositif de régulation, un dispositif de stimulation et un dispositif de réparation neuro-endovasculaire.
